# EUROPÄISCHE PATENTSCHRIFT

(11) **EP 0 264 952 B1**
(45) Veröffentlichungstag und Bekanntmachung des Hinweises auf die Patenterteilung: **15.09.1993**
(21) Anmeldenummer: 87115584.2
(22) Anmeldetag: 23.10.1987
(51) Int. Cl.: A61F 13/15

(54) **Verfahren zum Herstellen von auf dem Körper zu tragenden Artikeln**
Process for manufacturing articles to be worn on the body
Procédé de fabrication d'articles au contact du corps

(30) Priorität: 24.10.1986 JP 253496/86; 20.08.1987 JP 207070/87
(43) Veröffentlichungstag der Anmeldung: 27.04.1988
(73) Patentinhaber: UNI-CHARM CORPORATION, Kawanoe-shi Ehime-ken (JP)
(72) Erfinder: Suzuki, Migaku, Kawanoe-shi Ehime-ken (JP); Ochi, Mitsuzo, Uma-gun Ehime-ken (JP); Kudo, Takeshi, Ehime-ken (JP)
(74) Vertreter: Sperling, Rüdiger, Dipl.-Ing.

(56) Entgegenhaltungen:
- GB-A- 216 059
- US-A- 3 860 003
- US-A- 4 597 760

## Beschreibung

Die Erfindung betrifft ein Verfahren zum Herstellen von auf dem Körper im Bereich beider Schenkeln eines Trägers zu tragenden Artikeln, insbesondere ein Verfahren zum Herstellen einer Wegwerfwindel oder einer Bedeckung einer Wegwerfwindel und ganz besonders ein Verfahren zum Einarbeiten elastischer Teile in einen solchen Artikel, um eine gewünschte Anpassung um die Schenkel z.B. eines Babys zu erreichen.

Es sind Wegwerfwindeln bekannt, bei denen in einander gegenüberliegenden Seitenklappen elastische Teile angeordnet sind, um einen Paßsitz um die Schenkel eines Trägers zu gewährleisten.

In einer aus der US-A-45 97 760 bekannten Windel dieser Art werden die elastischen Teile üblicherweise auf einander gegenüberliegenden Seiten der Windel parallel zueinander angeordnet.
Aus der GB-A-2 161 059 - sie liegt dem Oberbegriff des Anspruchs 1 zugrunde - ist eine Windel bekannt, bei der die innere flüssigkeitsdurchlässige Lage zwei parallel laufende, sich von dem Bauchbereich bis zu dem Gesäßbereich erstreckende Falten aufweist, in deren obersten Faltenrücken ein Gummifaden eingearbeitet ist. Beim Anlegen richten sich diese Falten auf und bilden im Zusammenwirken mit dem Körper einen abgeschlossenen Bereich, in welchem feste Ausscheidungen gehalten werden, ohne daß sie an der Beinöffnung austreten können. Diese Anordnung hat den Nachteil, das die aufgerichteten Falten genau über das Gesäß laufen, so daß sie bei einem sitzenden Kind zum einen verdrückt werden, und so möglicher Weise ihre Funktion nicht mehr ausüben können, und zum anderen dem Kind ein unangenehmes Gefühl geben können.

Um den Paßsitz der Windel um die Schenkel eines Trägers zu verbessern, ist es erwünscht, daß der Abstand zwischen den gegenüberliegenden elastischen Teilen im Gesäßbereich (im folgenden stets: rückwärtigen Bereich) weiter ist als im Bauchbereich (im folgenden stets : vorderen Bereich). Eine Windel, die dieses Erfordernis erfüllt, ist ebenfalls bekannt. Des weiteren sind Windeln vom sogenannten aufgedrehten Typ (Turn-Up-Typ) bekannt, bei denen die einander gegenüberliegenden Seiten, versehen mit zugeordneten elastischen Teilen, nach innen gefaltet und anschließend die Seitenklappen an mittleren Abschnitten ihrer Länge mit der oberen Fläche der oberen Lage im Bereich des Gebiets verbunden werden, der den Saugkern bedeckt, so daß die einander gegenüberliegenden Teile von den miteinander verbundenen Abschnitten in Richtung der Vorder- und der Rückseite der Windel divergieren.

Bei einer solchen Windel, die aus Wettbewerbsgründen ein Massenprodukt mit minimalen Kosten sein sollte, ist es schwierig, einen wirtschaftlichen Herstellungsprozeß zu etablieren, der es gestattet, den Abstand zwischen den einander gegenüberliegenden elastischen Teilen graduell zu variieren. Üblicherweise werden verschiedene Komponenten der Windel und elastischen Teile kontinuierlich der Produktionslinie der Windel zugeführt, während das elastische Teil mittels eines Klebstoffs an den Komponenten befestigt wird.

Es ist dabei schwierig, die Bewegungsrichtung der elastischen Teile für jede der einzelnen Windeln in einer besonderen Zone der Windel zu ändern. Selbst wenn diese Schwierigkeiten überwindbar werden sollten, so können doch diese Versuche nicht das Erfordernis einer Hochgeschwindigkeitsproduktion erfüllen, wobei derartige Versuche wahrscheinlich zusätzliche Schwierigkeiten hervorrufen würden. Aus diesen Gründen wurden bisher, wie oben beschrieben, die einander gegenüberliegenden elastischen Teile der Windel des bezeichneten Typs üblicherweise in der Windel parallel zueinander angeordnet. Obwohl die angesprochene Turn-Up-Windel relativ leicht herzustellen ist, läßt jedoch der Paßsitz zu wünschen übrig.

Es ist die Aufgabe der Erfindung, ein verbessertes Verfahren zum Herstellen der Windeln anzugeben, das es ermöglicht, die einander gegenüberliegenden elastischen Teile so anzuordnen, daß der Abstand wahlweise variiert werden kann.

Die Aufgabe wird bei einem Verfahren zum Herstellen von auf dem Körper im Bereich beider Schenkeln eines Trägers zu tragenden Artikeln, wie beispielsweise eine Wegwerfwindel mit Seitenklappen an einander gegenüberliegenden Seiten und in den jeweiligen Seitenklappen angeordneten elastischen Teilen, die einem elastischen Paßsitz um die Schenkel eines Trägers dienen, erfindungsgemäß durch den Kennzeichnenden Teil des Anspruchs 1 gelöst.

Trotz der Tatsache, daß die gegenüberliegenden elastischen Teile zunächst in den jeweiligen zweiten Abschnitten parallel zueinander angeordnet werden, korrespondiert auf diese Weise der Abstand (d.h. der Winkel) zwischen ihnen mit demjenigen zwischen den Verbindungslinien, die vorher festgelegt sind, wenn die zweiten Abschnitte nach außen gewickelt werden. Während der Herstellung der Windel ist es demzufolge mögliche sicherzustellen, daß der Abstand zwischen den einander gegenüberliegenden elastischen Teilen zumindest in dem rückwärtigen Bereich weiter ist als im Schrittbereich, ohne daß, wie in der Diskussion erwähnt, komplizierte Mittel und Verfahren anzuwenden sind, um dieses Problem zu lösen. Die vorliegende Erfindung ermöglicht es, konventionelle Mittel und Verfahren anzuwenden, mittels denen die einander gegenüberliegenden elastischen Teile zunächst parallel zueinander angeordnet werden und die für eine Hochgeschwindigkeitsproduktion geeignet und im wesentlichen frei von Schwierigkeiten sind.

Im folgenden wird die Erfindung anhand in der Zeichnung dargestellter Ausführungsbeispiele näher erläutert. Es zeigen:
Figur 1 - 5 sequentiell ein Verfahren, in welchem ein erstes Ausführungsbeispiel einer erfindungsgemäßen Windel zusammengebaut wird, wobei:
   Figur 1A eine Draufsicht auf eine Windel zeigt, die auf einander gegenüberliegenden Seiten mit sich parallel zueinander erstreckenden elastischen Teilen versehen ist,
   Figur 1B einen schematischen Querschnitt entlang der Linie 12,
   Figur 2A eine Draufsicht auf dieselbe Windel, bei der einander gegenüberliegenden Seiten nach innen gefaltet worden sind,
   Figur 2B einen schematischen Querschnitt durch die Windel aus Fig. 2A entsprechend demjenigen aus Fig. 1B,
   Figur 3A eine Draufsicht auf dieselbe Windel, bei der die Außenränder entlang der Faltlinien teilweise verklebt worden sind,
   Figur 3B ein schematischer Querschnitt durch die Windel aus Fig. 3A entsprechend dem Querschnitt in Fig. 1B,
   Figur 4 eine Draufsicht auf dieselbe Windel mit weggeschnittenen, sich außerhalb der jeweiligen Kleblinien erstreckenden Bereichen,
   Figur 5 eine Draufsicht auf dieselbe Windel, bei der die gefalteten Bereiche nach außen zurückgeklappt worden sind,
Fig. 6-9 jeweils sequentielle Darstellungen eines Verfahrens, mit dem ein zweites Ausführungsbeispiel einer erfindungsgemäßen Windel zusammengebaut wird, wobei:
   Figur 6A eine Draufsicht auf einen Hauptkörper einer Windel zeigt , der an einander gegenüberliegenden Seiten mit Seitenklappen und separate Seitenklappenabschnitte versehen ist, die mit elastischen Teilen ausgestattet sind und mit dem Hauptkörper verbunden werden sollen,
   Figur 6B ein schematischer Querschnitt durch den Hauptkörper und die separaten Seitenklappen aus Fig. 6A,
   Figur 7A eine Draufsicht auf den Hauptkörper der Windel, wobei die separaten Seitenklappenabschnitte auf die entsprechenden Seitenklappen des Hauptkörpers aufgelegt sind,
   Figur 7B ein schematischer Querschnitt durch die Anordnung aus Fig. 7A,
   Figur 8 eine Draufsicht auf dieselbe Windel, wobei die gegenüberliegenden Seitenklappenbereiche entlang ihrer Außenkanten verklebt worden sind,
   Figur 9 eine Draufsicht auf dieselbe Windel, bei der die separaten Seitenklappenabschnitte nach außen aufgefaltet worden sind,
Figur 10 eine Draufsicht auf ein drittes Ausführungsbeispiel einer erfindungsgemäßen Windel im aufgeklappten Zustand,
Figur 11 eine perspektivische Ansicht derselben Windel, wobei sich die elastischen Teile in einem gewissen Ausmaß zusammengezogen haben,
Figur 12 und 13 jeweils Ansichten von Schnitten entlang der Linie XII-XII in Fig. 10,
Figur 14 - 21 Draufsichten verschiedener Arten von Anordnung einer Verbindungslinie auf jeder Seitenklappe und
   Figur 14A bis 14F, 17A und 17B, 18A und 18B
      Draufsichten, die die unterschiedliche Form zeigen, in welche ein freier Endabschnitt der Seitenklappe durch die Verbindungslinie gebogen oder gekrümmt wird.

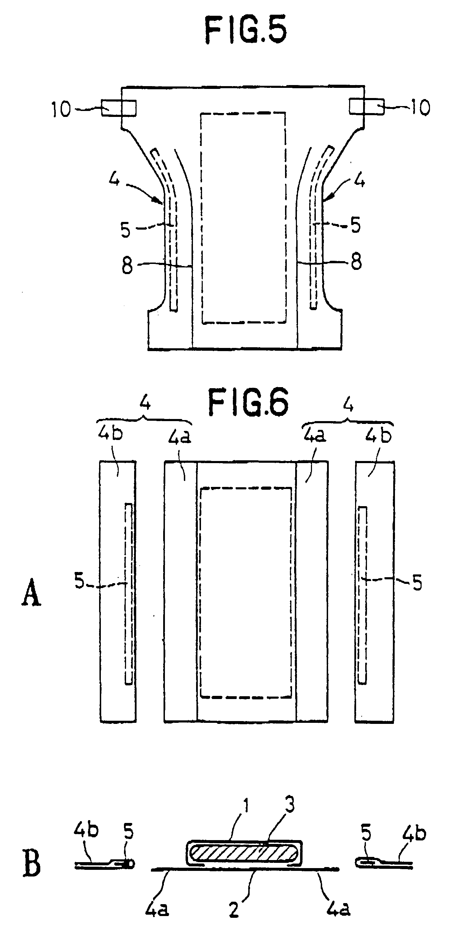

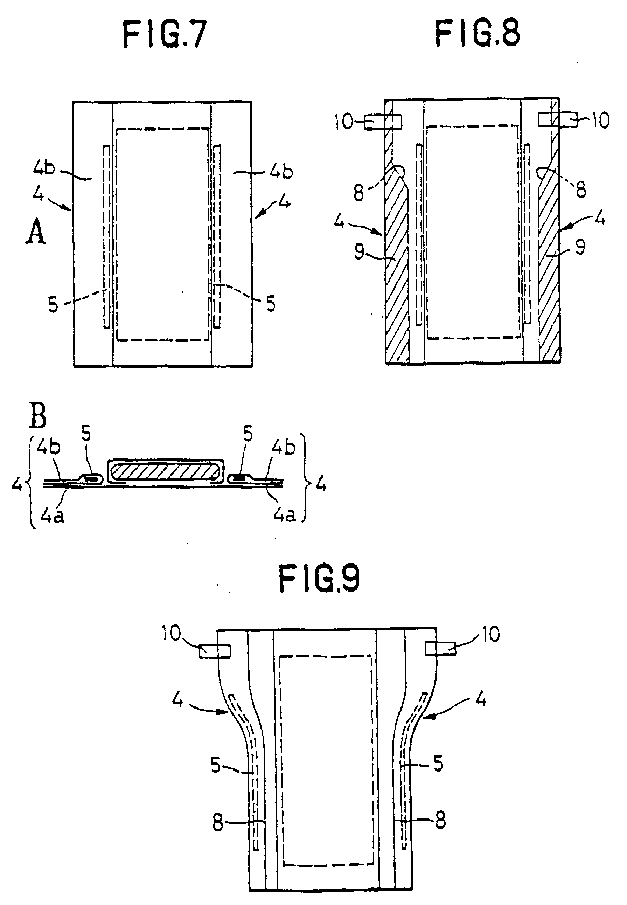

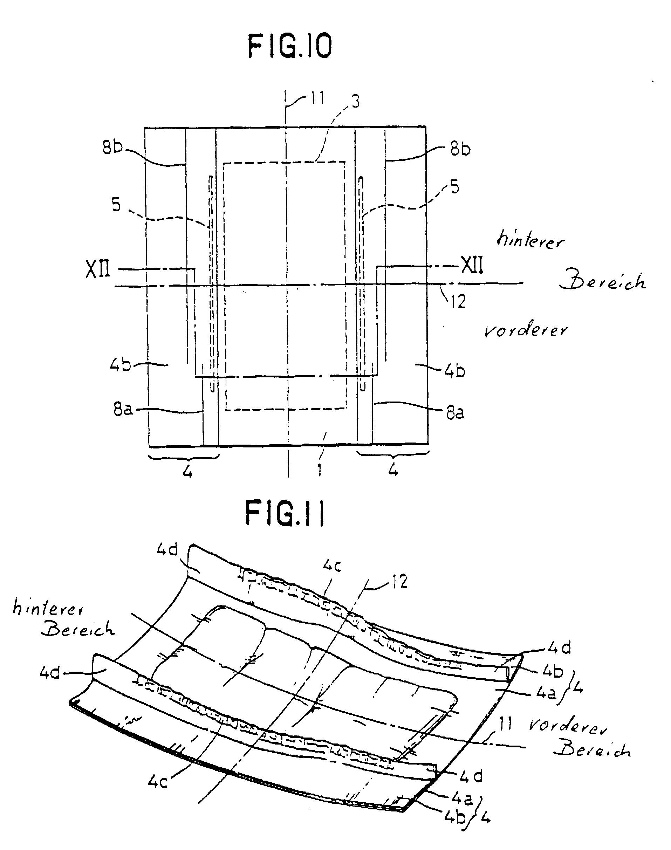

Im folgenden wird die Erfindung anhand einer Wegwerfwindel unter Bezugnahme auf die beigefügten Zeichnungen näher beschrieben.

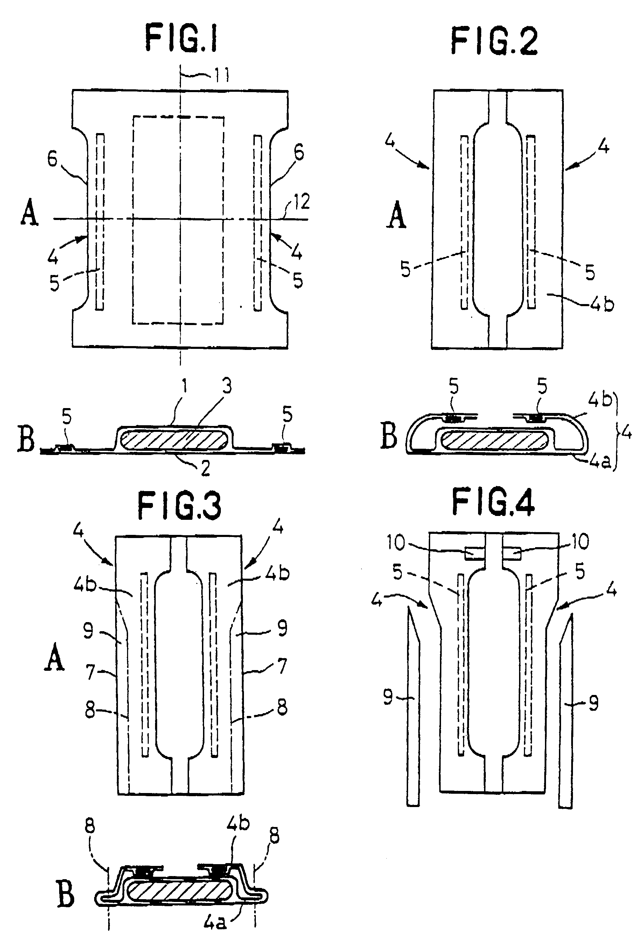

(A) Die Figuren 1 bis 5 zeigen ein erstes Ausführungsbeispiel der vorliegenden Erfindung.
   Wie in den Fig.1A und 1B dargestellt, ist ein Saugkern 3 sandwichartig zwischen einer wasserdurchlässigen oberen Lage 1 und einer wasserundurchlässigen äußeren Lage 2 eingeschlossen, wobei elastische Teile 5, die in Längsrichtung elastisch sind, parallel zueinander in jeweiligen Seitenklappen 4 eingearbeitet. Die Seitenklappen 4 werden durch Abschnitte der oberen Lage 1 und der äußeren Lage 2 gebildet, die sich von den einander gegenüberliegenden Seitenrändern des Saugkörpers 3 nach außen erstrecken. Im vorliegenden Fall sind die obere Lage 1, die äußere Lage 2 und der Saugkörper 3 mit einem heißschmelzenden Kleber miteinander verklebt,während die angesprochenen Abschnitte der oberen Lage 1 und der äußeren Lage 2, welche die Seitenklappen 4 bilden, mittels des bezeichneten Klebers auch mit dem elastischen Teil 5 verbunden sind. Einander gegenüberliegende Seiten der Seitenklappen 4 können teilweise weggeschnitten sein, um Ausschnittsbereiche 6 zu bilden. Es sei darauf hingewiesen, daß das Herstellen von Wegwerfwindeln des bisher beschriebenen Aufbaus ansich bekannt ist.
   Wie aus den Figuren 2A, 2B, 3A und 3B zu erkennen ist, werden anschließend die einander gegenüberliegenden Seitenklappen 4 des auf diese Weise hergestellten Windelrohteils nach innen gefaltet, um untere erste Abschnitte 4a, und obere zweite Abschnitte 4b zu bilden, wobei jeweils die zweiten Abschnitte 4b die ersten Abschnitte 4a überlagern. Anschließend werden die so zusammengefalteten ersten und zweiten Abschnitte 4a und 4b jeweils unisono entlang einander gegenüberliegender Verbindungslinien dera rt miteinander verklebt, so daß diese Verbindungslinien in einem rückwärtigen Bereich weiter voneinander entfernt sind, als in einem vorderen Bereich der Windel. Schließlich werden Abschnitte 9 der Windel, die sich jenseits der zugeordneten Verbindungslinien 8 erstrecken, weggeschnitten, um die Konturen der Windel auszubilden. Es sei hier darauf hingewiesen, daß die Abschnitte 9 auch belassen werden können, da diese Abschnitte keine unmittelbare Auswirkung auf die Funktion der Windel haben.
   Während des Gebrauchs der auf diese Weise hergestellten Windel werden die zweiten Abschnitte 4b der jeweiligen Seitenklappen 4 entlang der zugeordneten Verbindungslinie 8 nach außen gefaltet, wie dies in Fig. 5 dargestellt ist. In dieser dargestellten Konfiguration sind sowohl die Weite der Windel als auch ein Abstand zwischen den einander gegenüberliegenden elastischen Teilen 5 im rückwärtigen Bereich größer (dieser Bereich bedeckt die Hüften) als im vorderen Bereich (dieser Bereich bedeckt den Bauch).
(B) Die Figuren 6 bis 9 zeigen ein zweites Ausführungsbeispiel gemäß der vorliegenden Erfindung.
   Wie in den Figuren 6A und 6B zu erkennen ist, wird der Saugkörper 3 mit Ausnahme eines Zentralbereichs der unteren Fläche des Kerns 3 mit der wasserdurchlässigen oberen Lage bedeckt. Anschließend wird diese Anordnung auf einen Zentralbereich der Oberfläche der wasserundurchlässigen äußeren Lage 2 gelegt und integral mit dieser verbunden. Die ersten Abschnitte 4a der Seitenklappen werden bei diesem Ausführungsbeispiel durch Abschnitte der äußeren Lage gebildet, die sich von einander gegenüberliegenden Seitenrändern des Saugkörpers 3 jeweils nach außen erstrecken. Ein paar separat vorgesehener wasserundurchlässiger Lagen in Form relativ schmaler Streifen ist im Inneren mit den in Längsrichtung elastischen Teilen 5 versehen, wobei die jeweiligen Streifen entlang der zugeordneten elastischen Teile 5 jeweils umgefalzt sind und somit die separat vorgesehenen Seitenklappen die zweiten Abschnitte 4b bilden.
   Ausgehend von den Windelkomponenten, die in der beschriebenen Weise gebildet worden sind, werden die jeweiligen zweiten Abschnitte 4b, wie aus den Figuren 7A,7B und 8 zu erkennen ist, auf die entsprechenden ersten Abschnitte 4a gelegt, so daß sich die elastischen Teile 5 jeweils entlang der inneren Seiten erstrecken. Anschließend werden diese aufeinandergelegten ersten und zweiten Abschnitte 4a und 4b entlang der äußeren Seitenkanten integral derart miteinander verklebt, daß die Verbindungslinien, die sich entlang der Innenseiten der verklebten Bereiche erstrecken, im rückwärtigen Bereich weiter voneinander entfernt sind, als im vorderen Bereich der Windel, um die entsprechende Formation zu gewährleisten. Ebenso wie im Falle des ersten Ausführungsbeispiels können die sich außerhalb der inneren Verbindungslinien 8 befindenden Abschnitte 9 weggeschnitten oder belassen werden, je nach dem wie es gewünscht wird.
   Für den Gebrauch werden die zweiten Abschnitte entlang der zugeordneten inneren Verbindungslinien nach außen gefaltet, wie es in Fig. 9 dargestellt ist. In diesem Zustand ist die Breite der Windel ebenso wie der Abstand zwischen den einander gegenüberliegenden elastischen Teilen 35 im rückwärtigen Bereich, der die Hüften bedeckt, größer als im vorderen Bereich, der den Bauch bedeckt.
   Aus dem oben Gesagten ist zu ersehen, daß das erste und zweite Ausführungsbeispiel hinsichtlich des Gegenstands der Erfindung im wesentlichen identisch sind, da der einzige Unterschied zwischen den beiden Ausführungsbeispielen darin besteht, daß die zweiten Abschnitte 4b im ersten Ausführungsbeispiel nach innen gefaltet sind, während die zweiten Abschnitte 4b im zweiten Ausführungsbeispiel separat hergestellt auf die ersten Abschnitte 4a aufgelegt und mit ihnen verbunden sind, anstatt einstückig mit ersterer ausgebildet zu sein und auf letztere aufgefaltet zu werden.
(C) In den Figuren 10 bis 21 ist ein drittes Ausführungsbeispiel dargestellt. Dieses Ausführungsbeispiel enthält verschiedene Varianten und Änderungen.

Es wird Bezug genommen auf die Figuren 10 bis 12, in denen der Saugkörper 3 sandwichartig zwischen der wasserdurchlässigen oberen Lage 1 und der wasserundurchlässigen unteren Lage 2 eingeschlossen ist. Die in Längsrichtung elastischen Teile 5 sind in gegenseitiger paralleler Anordnung in den jeweiligen Seitenklappen eingearbeitet, die von sich von den einander gegenüberliegenden Längsrändern des Saugkörpers 3 nach außen erstreckenden Abschnitten der oberen Lage 1 und der unteren Lage 2 gebildet werden. Im vorliegenden Fall sind die obere Lage 1, die untere Lage 2 und der Saugkörper 3 mittels eines heißschmelzenden Klebers miteinander verbunden, wobei die bezeichneten Abschnitte der oberen Lage und der unteren Lage, die gemeinsam die Seitenklappen 4 bilden, ebenfalls mittels dieses Klebers mit dem elastischen Teil 5 verbunden sind.

Die einander gegenüberliegenden Seitenklappen 4 werden anschließend nach innen gefaltet, so daß die ersten unteren Abschnitte 4a und die oberen zweiten Abschnitte 4b, welche die ersten Abschnitte 4a jeweils überlagern, gebildet werden. Anschließend werden die übereinanderliegenden ersten und zweiten Abschnitte jeweils gemeinsam entlang innerer und äußerer Verbindungslinien 8a und 8b miteinander verbunden. Diese Verbindungslinien 8a und 8b erstrecken sich in Längsrichtung der Windel in den ersten und zweiten Abschnitten 4a und 4b außerhalb der elastischen Teile, die entlang der inneren Seitenkanten der zweiten Abschnitte 4b verlaufen. Auf diese Weise werden freie Endabschnitte gebildet, die sich von diesen Verbindungslinien weg erstrecken. Die jeweiligen inneren Verbindungslinien 8a sind relativ kurz und verlaufen auf der vorderen Hälfte des vorderen Bereichs der Windel während die äußeren Verbindungslinien 8b relativ lang sind und sich von hinteren Enden der jeweiligen inneren Verbindungslinien 8a bis zum hinteren Ende des rückwärtigen Bereichs der Windel erstrecken. Es sei so darauf hingewiesen, daß, obwohl diese Verbindungslinien 8a, 8b derart dargestellt sind, als wenn sie ausgeprägte V-förmige Querschnitte aufweisen, dies ist jedoch in Wirklichkeit nicht der Fall; die übertriebene Darstellungsform geschieht lediglich aus Deutlichkeitsgründen. Es versteht sich, daß nicht nur der Verbindungsprozeß unter Verwendung eines Klebstoffs sondern ebenfalls ein Schweißprozeß derartige übertriebenene Formen nicht hervorbringt.

Die vollständige, auf diese Weise hergestellte Windel besitzt rechte und linke Bereiche, die hinsichtlich einer zentralen Längsmittellinie 11 symmetrisch sind. Die vorderen und rückwärtigen Bereiche sind ebenfalls zu einer quer verlaufenden Mittellinie 12 symmetrisch.

In der Windel, die in der oben beschriebenen Weise geformt und dann entlang der inneren und äußeren Verbindungslinien 8a, 8b, wie in Fig. 10 dargestellt, zusammengeklebt worden ist, erheben sich die freien Endabschnitte 4c der zweiten Abschnitte 4b unter dem Einfluß eines Zusammenzieheffekts der elastischen Teile 5 entlang der Verbindungslinien 8a und 8b und zwar in dem Ausmaß, wie die elastischen Teile 5 sich zusammenziehen, vergl. Fig. 11. Der Abstand der auf diese Weise aufrecht stehenden freien Endabschnitte 4c der jeweiligen zweiten Abschnitte 4b voneinander ist im wesentlichen in der vorderen Hälfte des vorderen Bereichs enger und vergrößert sich graduell zum rückwärtigen Bereich der Windel.

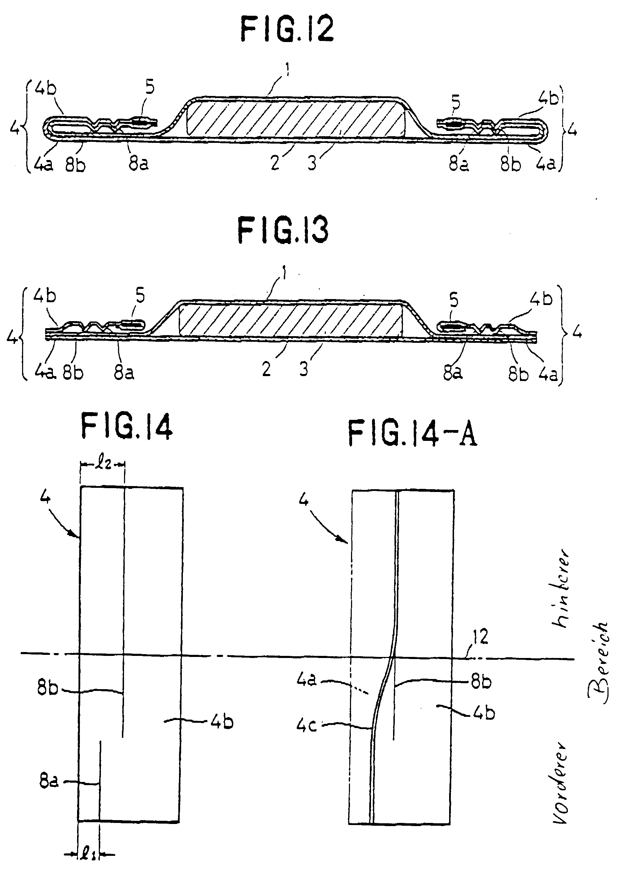

In Fig. 13 ist ein Ausführungsbeispiel dargestellt, bei welchem die zweiten Abschnitte 4b separat von den ersten Abschnitten 4a hergestellt und anschließend auf die entsprechend zugeordneten ersten Abschnitte 4a aufgelegt worden sind. Danach erfolgt das Verbinden dieser beiden Abschnitte entlang der inneren und äußeren Verbindlinien 8a, 8b. Es versteht sich, daß die vorliegende Erfindung nicht auf derartige Ausführungsbeispiele beschränkt ist, sie kann vielmehr wie in Fig. 12 dargestellt, solche Ausbildungsformen umfassen, bei welchen die jeweiligen Seitenklappen 4 teilweise auf sich selber gefalzt sind, um die jeweiligen zweiten Abschnitte 4b zu bilden.

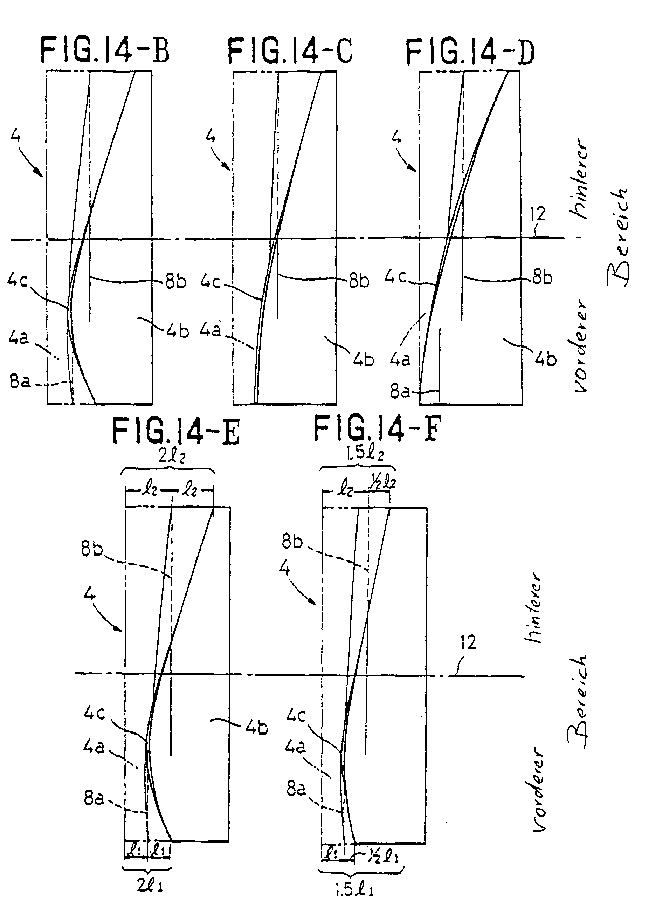

Im folgenden werden die Figuren 14 bis 21 erläutert:
(1) Es wird lediglich auf die Seitenklappe 4 des rechten Bereichs der Windel, wie sie in Fig. 10 dargestellt ist, bezug genommen; der linke Bereich ist entsprechend gestaltet und wird daher nicht gesondert erläutert .
(2) mit Verbindungslinien 8a und 8b sind diejenigen gemeint, die sich in Längsrichtung in den gemeinsam jede der Seitenklappen 4 bildenden Abschnitten 4a und 4b erstrecken und sie miteinander verbinden;
(3) in den in den Figuren 14A bis 14F, 17A,17B und 18A und 18B dargestellten Ausführungsbeispielen soll die Konfiguration, die die obere Kante des freien Endabschnitts 4c jedes zweiten Abschnitts 4 einnimmt, wenn der freie Endabschnitt entlang der Verbindungslinien 8a und 8b aufgerichtet ist, so verstanden werden, daß diese Konfiguration unter der Bedingung in dieser Form in Erscheinung tritt, daß das elastische Teil 5, das am freien Ende des Endabschnitts 4 angeordnet ist, in bestimmten Ausmaßen zusammengezogen ist, wie es in Verbindung mit Fig. 11 erläutert wurde,
(4) in Verbindung mit einer Beschreibung dieser Konfigurationen des freien Endabschnitts 4c entlang seiner oberen Kante (oben näher erläutert) zeigen doppelte Linien in jeder dieser Figuren, daß ein Bereich, der diesen doppelten Linien entspricht und ihnen benachbart ist, im wesentlichen in eine vertikale Richtung aufgerichtet ist, wohingegen eine einfache Linie anzeigt, daß ein Bereich, der dieser Einzellinie entspricht, in einem gewissen Maß von den Enden des Bereiches, der der doppelten Linie entspricht und benachbart dazu ist, schräg nach außen zum vorderen oder hinteren Ende dieses Bereiches, der der einzelnen Linie entspricht, geneigt ist und vollständig an diesen vorderen und hinteren Enden nach außen umklappt; und
(5) die Ausdrücke" vorderes Ende" und" hinteres Ende" des zweiten Abschnitts 4b sollten als ein Abschnitt verstanden werden, der dem Abschnitt 4d in Fig. 11 entspricht.

Fig. 14 zeigt eine Grundanordnung der Verbindungslinien in den Seitenklappen 4, die sicherstellt, daß der Abstand zwischen den gegenüberliegenden freien Endabschnitten 4c in den linken und rechten Bereichen der Windel 4c im wesentlichen in der vorderen Hälfte des vorderen Bereichs relativ eng und demgegenüber im wesentlichen in der hinteren Hälfte des vorderen Bereichs und des rückwärtigen Bereichs größer ist. In dieser Anordnung ist in dem vorderen Bereich der Windel und zwar in der vorderen Hälfte der Seitenklappe 4 eine relativ kurze innere Verbindungslinie 8a, in der hinteren Hälfte des vorderen Bereichs und im rückwärtigen Bereich der Windel hingegen eine relativ lange fortlaufende äußere Verbindungslinie 8b vorgesehen. Der freie Endabschnitt 4c, der durch das Verbinden ausgebildet wird, erhebt sich entlang der Verbindungslinien 8a und 8b; es ergibt sich eine obere Kante des freien Endabschnitts 4c, die sich wie in der Draufsicht der Fig. 14A zu erkennen ist, im wesentlichen in einer geschwungenen Konfiguration darstellt.
Mit einer Anordnung, wie sie in Fig. 14A dargestellt ist, erhält die obere Kante des freien Endabschnitts 4c, wenn das vordere und hintere Ende dieses Endabschnitts 4 C nach außen (in der Figur nach rechts) gefaltet und befestigt werden, eine konkav gekrümmte Konfiguration mit dem Krümmungsscheitel zum Windelinneren weisend, wie es in der Draufsicht der Figur 14B dargestellt ist. Wenn lediglich das hintere Ende nach außen gefaltet (in der Figur nach rechts) und dann befestigt wird, erhält die obere Kante eine im wesentlichen flache gekrümmte Form, wie sie in einer Draufsicht der Fig. 14C zu erkennen ist. Schließlich, wenn das vordere Ende nach innen (in der Figur nach links) gefalztet und dann befestigt wird, während das hintere Ende nach außen (in der Figur nach rechts) gefalztet und befestigt wird, so weist die obere Kante nur eine schwach gekrümmte Form auf, wie sie im wesentlichen aus der Draufsicht der Figur 14D zu entnehmen ist.

Bei einer Anordnung, wie sie in Fig. 14E gezeigt ist, wird der freie Endabschnitt 4c nahe dem vorderen Ende der inneren Verbindungslinie 8a über eine Breite von 2 1₁ gefaltet und befestigt, d.h. das doppelte der Breite 1₁, die sich von der inneren Seitenkante des zweiten Abschnitts 4b zur inneren Verbindungslinie 8a erstreckt, während das dem hinteren Ende der äußeren Verbindungslinie 8a zugewandte freie Ende des Abschnitts 4c über eine Breite 21₂ gefaltet und befestigt wird, d.h. das Doppelte der Breite 1₂, die sich von der inneren Seitenkante des zweiten Abschnitts 4b bis zur äußeren Verbindungslinie 8b erstreckt. Bei der Anordnung, die in Fig. 14F dargestellt ist, wird der freie Endabschnitt in der Nachbarschaft des vorderen Endes der inneren Verbindungslinie 6a über eine Breite von 1,5 1₂ gefaltet und befestigt, d.h. eineinhalb Mal die Breite 1₁, die sich von der inneren Seitenkante des freien Endabschnitts 4c zur inneren Verbindungslinie 8a erstreckt, während demgegenüber am Ende der äußeren Verbindungslinie 8b dieses Falten und Befestigen des Abschnitts 4c mit einer Breite von 1,5 1₂ durchgeführt wird, d.h. eineinhalb Mal eine Breite, die sich von der inneren Seitenkante des zweiten Abschnitts 4b zur äußeren Verbindungslinie 8b erstreckt, so daß der Krümmungsradius des freien Endabschnitts in der Anordnung der Figur 14F größer ist, als derjenige der Anordnung gemäß 14E.

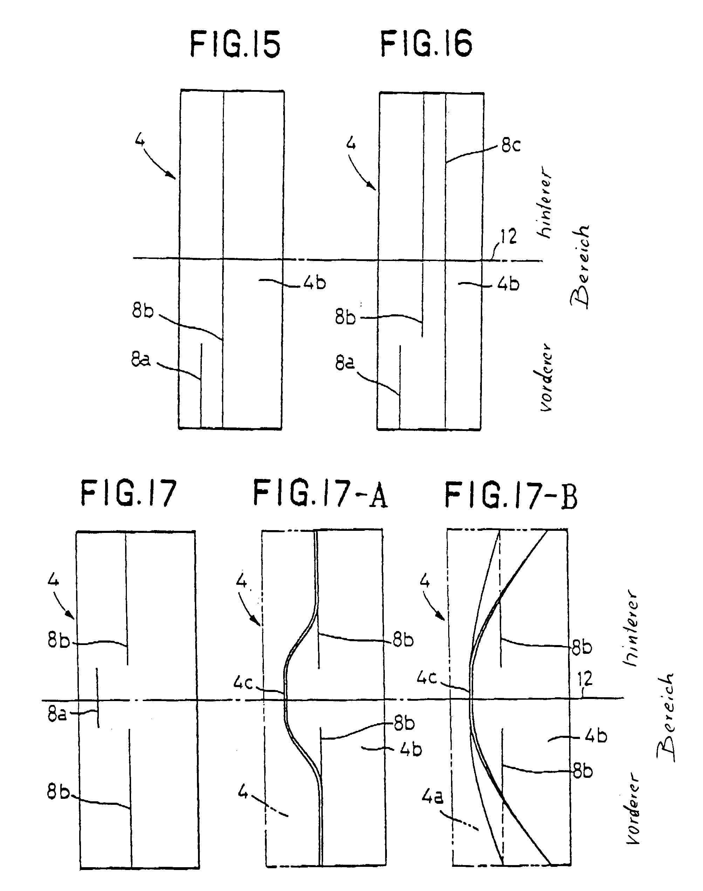

Die Anordnung aus Fig. 15 unterscheidet sich von der Anordnung aus Fig. 14 dadurch, daß die äußere Verbindungslinie 8b zum vorderen Ende des vorderen Bereichs verlängert ist, so daß sie sich kontinuierlich über die gesamte Länge der vorderen und rückwärtigen Bereiche erstreckt. In der Anordnung der Figur 16 ist eine dritte äußerste Verbindungslinie 8c vorgesehen, die sich kontinuierlich über die gesamte Länge des vorderen und rückwärtigen Bereichs ersteckt, zusätzlich zu den Verbindungslinien 8a und 8b der Anordnung der Figur 14. Das Anbringen solcher kontinuierlicher Verbindungslinien 8c ist für den Fall geeignet, bei dem, wie bereits in Verbindung mit der Anordnung aus Fig. 13 erläutert, die zweiten Abschnitte 4b getrennt von den ersten Abschnitten 4a ausgebildet werden, so daß erstere jeweils auf letztere aufgelegt werden. Selbst wenn die Bereiche dieser ersten und zweiten Abschnitte, die sich außerhalb der Verbindungslinien erstrecken, nicht wasserdicht miteinander verbunden sind, so verhindern diese kontinuierlichen Verbindungsschichten mit guter Wirkung, daß die aufgesaugte Körperflüssigkeit im Saugkern 3 durch die Schichten des ersten Abschnitts 4a und des zweiten Abschnitts 4b nach außen fließen kann.

Figur 17 zeigt eine Grundanordnung von Verbindungslinien in den jeweiligen Seitenklappen der Windel, womit sichergestellt wird, daß der Abstand zwischen den seitlichen, einander gegenüberliegenden freien Enden der Abschnitte 4c in einem Zentralbereich, bezogen auf eine imaginäre Quermittellinie 12 kleiner ist als in den vorderen und rückwärtigen Bereichen. Bei dieser Anordnung wird eine relativ kurze innere Verbindungslinie 8a so angeordnet, daß diese die imaginäre quer verlaufende Mittellinie 12 etwa an einem Mittelpunkt der Verbindungslinie 8a schneidet, während zwei relativ lange äußere Verbindungslinien 8b jeweils in den vorderen und rückwärtigen Bereichen angeordnet sind. Der auf diese Weise gebildete freie Endabschnitt 4c erhebt sich entlang der Verbindungslinien 8a und 8b, wobei die obere Randkante eine gekrümmte Ausbildung hat, wie sie in Fig. 17A zu erkennen ist, d.h. die gerade verlaufende Rippe weist auf Höhe der querverlaufenden Mittellinie eine zum Saugkern gerichtete konvexe Form auf.

Aus dem Zustand, wie er in Fig.17A dargestellt ist, werden die vorderen und rückwärtigen Enden des freien Endabschnitts 4c nach außen gefaltet und festgelegt so, daß die obere Kante des freien Endabschnitts 4c eine Konfiguration aufweist, wie sie in Fig. 17B dargestellt ist, d.h. in Richtung zum Saugkern bildet die obere Randkante eine konvexe Kurve mit dem Scheitel in der Nähe des Saugkerns.

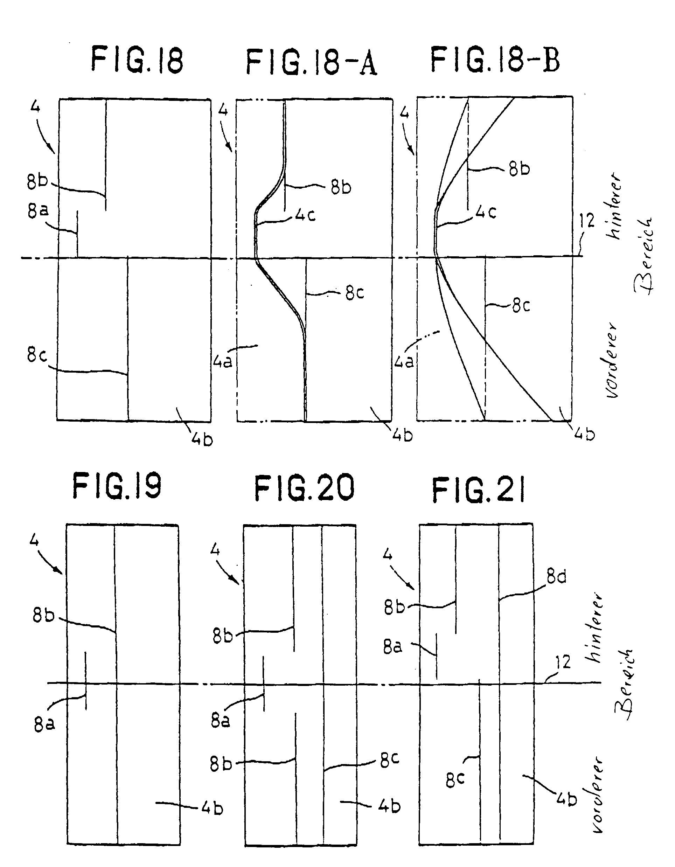

Die in Fig. 18 dargestellte Anordnung entspricht der Anordnung der Fig. 17, ist jedoch geringfügig geändert. In dieser geänderten Anordnung ist eine relativ kurze innere Verbindungslinie 8a und eine relativ lange äußere Verbindungslinie 8b im rückwärtigen Bereich der Windel vorgesehen, während eine längere äußerste Verbindungslinie 8c in dem vorderen Bereich vorgesehen ist. Der auf diese Weise gebildete freie Endabschnitt 4 c erhebt sich im wesentlichen in einer Vertikalrichtung entlang der jeweiligen Verbindungslinien 8a,b und c wobei die obere Randkante eine Konfiguration aufweist, wie sie aus Fig. 18A zu erkennen ist. Aus dieser in Fig. 18 dargestellten Zwischenanordnung werden die vorderen und hinteren Enden des freien Endabschnitts 4c nach außen gefaltet und befestigt, so daß die obere Randkante des freien Endabschnitts 4c eine gekrümmte Konfiguration aufweist, die in der Draufsicht der Fig. 18B dargestellt ist, d.h. im Schrittbereich erhebt sich dieser freie Endabschnitt 4c im wesentlichen senkrecht, während die jeweiligen Endabschnitte sich der jeweiligen Seitenklappenlage nähern, wobei die obere Kante des freien Endabschnitts wiederum bezüglich des Saugkörpers eine konkave Kurve mit Scheitel in der Nähe des Saugkörpers beschreibt, wobei jedoch der Ast der Kurve, der sich im rückwärtigen Bereich befindet, flacher ist als derjenige im vorderen Bereich der Windel.

In der Anordnung aus Fig. 19 wurden die äußeren unterbrochenen Verbindungslinien 8b der Anordnung aus Fig. 17 miteinander verbunden,so daß hier eine einzige kontinuierliche Verbindungslinie vorliegt. Figur 20 zeigt eine Anordnung, in der zusätzlich zu den Verbindungslinien 8a und 8b sich die äußerste Verbindungslinie 8c kontinuierlich über die gesamte Länge des vorderen und des rückwärtigen Bereichs der Windel erstreckt. Schließlich zeigt Fig. 21 eine Anordnung, in der zusätzlich zu den Verbindungslinien 8a, 8b und 8c der Anordnung gemäß Fig. 18 eine weitere äußere Verbindungslinie 8d vorgesehen ist, die sich über die gesamte Länge des vorderen und des rückwärtigen Bereichs der Windel erstreckt. Der Grund für das Vorsehen solcher kontinuierlicher Verbindungslinien 8b (Fig. 19), 8c (Fig. 20) oder 8d (Fig. 21), die sich über die gesamte Länge der vorderen und rückwärtigen Bereiche der Windel erstrecken, ist der gleiche, wie im Zusammenhang mit den Figuren 15 und 16 beschrieben.
Wie aus den oben in Verbindung mit den jeweiligen Figuren kurz erläuterten verschiedenen Anordnungen zu ersehen, ist es für die Erfindung wesentlich, daß zumindest innere und äußere Verbindungslinien 8a und 8b vorgesehen werden, um den ersten Abschnitt 4a und den zweiten Abschnitt 4b miteinander zu verbinden, welche Abschnitte die Seitenklappen 4 bilden. Es ist ebenso wesentlich, daß der Abstand zwischen der äußeren Kante des Saugkörpers 3 und dem freien Endabschnitt 4c in dem Bereich, der keine innere Verbindungslinie 8a enthält und der zwischen den inneren Kanten des zweiten Abschnitts 4b, der äußeren Verbindungslinie 8b und deren gedachten Verlängerung liegt, relativ groß ist. Des weiteren ermöglicht das Anordnen zumindest innerer und äußerer Verbindungslinien 8a und 8b, daß wunschgemäß elastische Linien zumindest teilweise auf dem jeweiligen freien Endabschnitt 4c unter Wirkung der jeweiligen elastischen Teile gebildet werden können. Zusätzlich können Faktoren wie Position, Winkel und Orientierung, gemäß welchen jede elastische Linie gebogen oder gekrümmt werden soll,durch genaues Auswählen der Länge und des Abstands zumindest der inneren und äußeren Verbindungslinie 8a und 8b bestimmt werden, unabhängig davon, ob der freie Endabschnitt 4c nach innen oder außen, und befestigt oder nicht befestigt wird, so daß demzufolge eine Kombination eines Nachinnenfaltens oder Nachaußenfaltens einer vollständigen oder teilweisen Befestigung der gefalteten Abschnitte möglich ist (vergl. auch Figuren 14E und 14F).

Es sei hier darauf hingewiesen, daß das gewünschte Befestigen mittels einer Vielzahl von Verbindungslinien, (einschließlich kurzer und langer Verbindungslinien oder eine Kombination dieser beiden Arten) oder einer breiten bandähnliche Verklebung, soweit es den Bereich betrifft, der sich außerhalb der äußeren Verbindungslinie 8b in den Figuren 14A,15,17 und 19 oder der äußersten Verbindungslinie 8c,8d in den Figuren 16,18,20 und 21, durchgeführt werden kann.
Diese Art der Befestigung wird insbesondere dann bevorzugt, wenn die zweiten Abschnitte 4b getrennt von den ersten Abschnitten 4a hergestellt werden, um anschließend jeweils auf letztere aufgelegt zu werden, so wie es in Fig. 13 dargestellt ist. Die Verbindungslinien 8a,8b...können als eine Kettenlinie (d.h. unterbrochene Linie) ausgeführt werden, u. zwar in dem Ausmaß, als die gewünschte Funktion erfüllt wird.

Die obere Lage 1 kann aus einem Faservlies oder einem porösen Kunststoffilm, die äußere Lage 2 vorzugsweise aus einem luftdurchläßigen Kunststoffilm oder einer Laminatlage aus einem solchen Film und einem Faservlies hergestellt sein, und der Saugkern 3 hauptsächlich aus einer flockigen Pulpe, vorzugsweise einer Pulpe, die aus einer Mischung hochapsorptiver polymerer Partikel gebildet wird, bestehen.Der zweite Endabschnitt 4b des zweiten Ausführungsbeispiels kann aus einem luftdurchläßigen Kunststoffilm, einem Laminat aus einem solchen Film und einem Faservlies oder luftdurchläßigem, nicht gewebtem Material bestehen, daß mit einem geeigneten Repellenz behandelt wurde.

Die zweiten Abschnitte 4b werden unter der zusammenziehenden Wirkung der jeweiligen Elastikteile 5 auf den Linien 8, entlang welchen die Seitenabschnitte 4b mit den zugeordneten ersten Abschnitten 4a verklebt sind, aufgerichtet und so angepaßt, daß sie während des Anlegens und des Gebrauchs der Windel nach innen fallen. Dementsprechend wird zusätzlich oder in Verbindung mit der Wirkung der vorliegenden Erfindung, wie sie oben beschrieben worden ist, ein zusätzlicher Effekt eines engen Paßsitzes um den Schenkel des Trägers bewirkt. Der Vollständigkeit halber sei noch erwähnt, daß der rückwärtige oder Hüftbereich der Windel an den einander gegenüberliegenden Seiten mit Bandbefestigungsmittel versehen ist.

Es sei darauf hingewiesen, daß das Verbinden ein Kleb-, ein Schweiß- oder ein beliebiger anderer geeigneter Verbindungsprozeß sein kann.

## Patentansprüche

1. Verfahren zum Herstellen von auf dem Körper im Bereich beider Schenkeln eines Trägers zu tragenden Artikeln, insbesondere Wegwerfwindel, mit einem vorderen, einem rückwärtigen und einem zwischen beiden liegenden Schrittbereich, mit am Rand des Artikels verlaufenden Seitenklappen und elastischen Teilen, die derart den jeweiligen Seitenklappen zugeordnet sind, daß sie darin elastische Linien bilden, um einen Paßsitz um die Schenkel des Trägers zu gewahrleisten, **gekennzeichnet** durch die folgenden Verfahrensschritte:
a) Aufteilen jeder Seitenklappe in einen ersten und einen zweiten, sich jeweils in Längsrichtung erstreckenden Abschnitt,
b) Anbringen des elastischen Teils auf den zweiten Abschnitt entlang des Seitenrandes, so daß sich das elastische Teil nach
c) Auflegen des zweiten Abschnitts auf den ersten Abschnitt entlang der Innenseite des zweiten Abschnitts erstreckt,
d) selektives Verbinden des ersten und zweiten Abschnitts innerhalb eines Bereichs, der zwischen ihrem gemeinsamen Außenrand und dem elastischen Teil liegt, entlang mindestens einer kontinuierlich durchgehenden und/oder mindestens einer nur über eine Teilstrecke vorgesehenen Verbindungslinie, wobei dem vorderen bzw. dem rückwärtigen Bereich jeweils mindestens eine sich in Längsrichtung erstreckende vordere bzw. hintere Verbindungslinie zugeordnet ist, und wobei die hinteren Verbindungslinien der Seitenklappen bestimmungsgemäß einen größeren oder einen geringeren Abstand voneinander aufweisen als die vorderen Verbindungslinien.

2. Verfahren nach Anspruch 1, dadurch **gekennzeichnet,** daß die Teilstrecken der Verbindungslinien zueinander seitlich versetzt sind.

3. Verfahren nach Anspruch 2, dadurch **gekennzeichnet,** daß zwei der Teilstrecken miteinander fluchten.

4. Verfahren nach einem der vorangegangenen Ansprüche, dadurch **gekennzeichnet,** daß die zweiten Abschnitte der Seitenklappen durch Falten eines Außenabschnitts der Seitenklappe nach innen erhalten wird.

5. Verfahren nach einem der vorangegangenen Ansprüche, dadurch **gekennzzeichnet,** daß die zweiten Abschnitte der Seitenklappen separat ausgebildet und anschließend mit den ersten Abschnitten der Seitenklappen, die sich von einander gegenüberliegenden Seiten des Artikels nach außen erstrecken, verbunden werden.

6. Verfahren nach einem der vorangegangenen Ansprüche, dadurch **gekennzeichnet,** daß das selektive Verbinden derart durchgeführt wird, daß sich von den Verbindungslinien nach außen freie Endabschnitte des zweiten Abschnitts erstrecken, die die elastischen Teile enthalten, wobei zwischen den versetzt angeordneten Teilen der Verbindungslinien keine Klebeverbindung besteht und wobei die elastischen Teile zumindest über einen Teil des freien Endabschnitts eine elastische Linie ausbilden.

7. Verfahren nach nach einem der vorangegangenen Ansprüche, dadurch **gekennzeichnet,** daß die Teilstrecken der Verbindungslinien im Schrittbereich einen geringeren Abstand voneinander aufweisen als die Verbindungslinien im vorderen bzw. rückwärtigen Bereich.

8. Verfahren nach einen der vorangegangenen Ansprüche dadurch **gekennzeichnet,** daß ein hinteres Ende des freien Endabschnitts nach außen gefaltet und befestigt wird.

9. Verfahren nach einem der vorangegangenen Ansprüche dadurch **gekennzeichnet,** daß ein vorderes Ende des freien Endabschnitts nach außen gefaltet und befestigt wird.

10. Verfahren nach einem der vorangegangenen Ansprüche, dadurch **gekennzeichnet,** daß sowohl das vordere als auch das hintere Ende des freien Endabschnitts nach außen gefaltet und befestigt wird.

11. Verfahren nach einem der vorangegangenen Ansprüche, dadurch **gekennzeichnet,** daß das vordere Ende des freien Endabschnitts nach innen und das hintere Ende nach außen gefaltet und befestigt wird.

12. Verfahren nach einem der vorangegangenen Ansprüche, dadurch **gekennzeichnet,** daß der auf dem Körper zu tragende Artikel eine Bedeckung einer Wegwerfwindel ist.

## Claims

1. Process for the manufacture of an article to be worn on the body of a wearer in the region of both thighs, in particular a disposable nappy, with a fore part a rear part and a crotch part lying between the two, with side flaps extending along the edge of the article and elastic parts which are arranged with respect to the respective side flaps such that they form elastic lines therein in order to ensure a good fit to the thigh of the wearer,
characterised by the following process steps:
a) dividing each side flap into a first and a second portion, each extending in the longitudinal direction,
b) applying the elastic part onto the second portion along the side edge so that
c) after laying the second portion onto the first portion the elastic part extends along the inner side of the second portion,
d) selective connection of the first and second portions within a region which lies between their mutual outer edge and the elastic part, along at least one continuous connection line and/or at least one partially formed connection line, whereby at least one fore or rear connection line, extending in the longitudinal direction, is arranged in each of the fore or the rear region, and whereby the rear connection lines of the side flaps have a larger or a lesser separation from each other as the fore connection lines.

2. Process according to Claim 1, characterised in that the partially formed connection lines are laterally displaced from one another.

3. Process according to Claim 2, characterised in that two of the partially formed connection lines are aligned with each other.

4. Process according to one of the preceding Claims, characterised in that the second portions of the side flaps are obtained by folding an outer portion of the side flap inwards.

5. Process according to one of the preceding Claims, characterised in that the second portions of the side flaps are formed separately and subsequently connected to the first portions of the side flaps, which extend outwards from opposite sides of the article.

6. Process according to one of the preceding Claims, characterised in that the selective connection is carried out in such a way that free end portions of the second portion extend outwards from the connection lines, the end portions including the elastic parts, whereby there is no adhesive connection between the displaced parts of the connection lines and whereby the elastic parts form an elastic line over at least part of the free end portion.

7. Process according to one of the preceding Claims, characterised in that the parts of the connection lines in the crotch region have a lesser separation from each other as the connection lines in the fore or rear parts.

8. Process according to one of the preceding Claims, characterised in that a rear end of the free end portion is folded outwards and secured.

9. Process according to one of the preceding Claims, characterised in that a forward end of the free end portion is folded outwards and secured.

10. Process according to one of the preceding Claims, characterised in that both the fore and the rear end of the free end portion is folded outwards and secured.

11. Process according to one of the preceding Claims, characterised in that the forward end of the free end portion is folded inwards and the rear end is folded outwards and secured.

12. Process according to one of the preceding Claims, characterised in that the article to be worn on the body is a cover of a disposable nappy.

## Revendications

1. Procédé pour la fabrication d'articles à porter au contact du corps, dans la zone de deux branches d'un support, de couchés jetables notamment, comportant une partie avant, une partie arrière et un entrejambe entre les deux, comportant sur le bord de l'article des pattes latérales et des éléments élastiques, qui sont associés aux pattes latérales respectives, de manière à former là des lignes élastiques, pour assurer un ajustage autour des branches du support, caractérisé par les étapes suivantes :
a) partage de chaque patte latérale en un premier segment et en un deuxième segment s'étendant respectivement en direction longitudinale,
b) mise en place de l'élément élastique sur le deuxième segment le long du bord latéral, de manière que l'élément élastique, après
c) application du deuxième segment sur le premier segment, s'étende le long de la face interne du deuxième segment,
d) raccordement sélectif des premiers et deuxièmes segments à l'intérieur d'une zone, qui se situe entre leur bord externe commun et l'élément élastique, au minimum le long d'une ligne de raccordement continue et/ou au moins le long d'une ligne de raccordement, qui n'est prévue que sur une section, tandis qu'à la partie avant et à la partie arrière sont respectivement associées au moins une ligne de raccordement avant ou une ligne de raccordement arrière s'étendant en direction longitudinale et cependant que les lignes de raccordement arrière des pattes latérales, en fonction de leur spécification, présentent, par rapport aux lignes de raccordement avant, un écartement plus grand ou plus petit entre elles.

2. Procédé selon la revendication 1, caractérisé en ce que les sections des lignes de raccordement sont décalées latéralement entre elles.

3. Procédé selon la revendication 2, caractérisé en ce que deux des sections sont alignées entre elles.

4. Procédé selon l'une des revendications précédentes, caractérisé en ce que le deuxième segment des pattes latérales est maintenu par repliement vers l'intérieur d'un segment externe de la patte latérale.

5. Procédé selon l'une des revendications précédentes, caractérisé en ce que le deuxième segment des pattes latérales est réalisé séparément, puis est raccordé au premier segment des pattes latérales, qui s'étend à partir des côtés opposés de l'article vers l'extérieur.

6. Procédé selon l'une des revendications précédentes, caractérisé en ce que le raccordement sélectif est réalisé de manière que les extrémités libres du deuxième segment, qui renferment les éléments élastiques, s'étendent à partir des lignes de raccordement vers l'extérieur, tandis qu'il n'existe pas de raccordement par colle entre les parties décalées des lignes de raccordement et cependant que les éléments élastiques constituent, sur une partie au moins de l'extrémité libre, une ligne élastique.

7. Procédé selon l'une des revendications précédentes, caractérisé en ce que les sections des lignes de raccordement présentent, dans le secteur de l'entrejambe, un écartement entre elles plus faible que celui des lignes de raccordement avant et arrière.

8. Procédé selon l'une des revendications précédentes, caractérisé en ce qu'une extrémité arrière du segment libre d'extrémité est repliée et fixée vers l'extérieur.

9. Procédé selon l'une des revendications précédentes, caractérisé en ce qu'une extrémité avant du segment libre d'extrémité est repliée et fixée vers l'extérieur.

10. Procédé selon l'une des revendications précédentes, caractérisé en ce que aussi bien l'extrémité avant que l'extrémité arrière du segment libre d'extrémité sont repliées et fixées vers l'extérieur.

11. Procédé selon l'une des revendications précédentes, caractérisé en ce que l'extrémité avant du segment libre d'extrémité est repliée et fixée vers l'intérieur et l'extrémité arrière vers l'extérieur.

12. Procédé selon l'une des revendications précédentes, caractérisé en ce que l'article à porter au contact du corps recouvre une couche jetable.
